# EUROPEAN PATENT APPLICATION

(11) **EP 0 744 179 A1**
(43) Date of publication of application: **27.11.1996**
(21) Application number: 95936767.3
(22) Date of filing: 10.11.1995
(51) Int. Cl.: A61M 1/00

(54) **DEFECATION DISPOSAL DEVICE**

(30) Priority: 11.11.1994 JP 277773/94
(71) Applicant: Sumitomo Bakelite Company Limited, Shinagawa-ku, Tokyo 140 (JP)
(72) Inventor: YAGATA, Kazuhiko, Akita-shi Akita 011 (JP); HIGUMA, Masato, Akita-shi Akita 011 (JP)
(74) Representative: Vossius, Volker, Dr.
(86) International application number: JP9502298
(87) International publication number: WO9614888

(57) **Abstract**

A cylindrical-shaped insert (3) comprises an irrigation nipple (5) on a side thereof and a suction nipple (6) at one end thereof, and is diverged at the other end thereof which is to be inserted into an anus. An enema liquid nozzle (4) is inserted into the insert (3) through the irrigation nipple (5). The enema liquid nozzle (4) is supplied with an enema liquid by an enema liquid pump (7) through an enema liquid tube (15). The enema liquid nozzle (4) is capable of jetting the enema liquid in all directions. The suction nipple (6) is connected to a storage vessel (11) through a suction tube (14). Excrements are drawn toward a diverged end of the insert (3) by a negative pressure produced by a suction pump (8) and are crushed by the enema liquid jetted from the enema liquid nozzle (4). Excrements thus crushed are stored in the storage vessel (11) together with the enema liquid by means of the suction pump (8).

## Description

### TECHNICAL FIELD

The present invention relates to an excretion treatment apparatus.

### BACKGROUND ART

To excrete feces, excretion is conventionally prompted by a laxative, an enema or taking-out of feces. In particular, excretion is forcibly effected to a patient exhibiting a dyschezia symptom due to hard feces by combining the above treatments. However, these treatments physically and mentally give violent pain to a person being treated (patient). In addition, a treating person must be engaged in an excretion treatment for a long time and further endure an offensive odor of feces.

The following treatments are known as means for solving the above problem in excretion. That is, one of them is that a balloon catheter is inserted into the anus and warm water is jetted to feces in the rectum so that the feces are broken and emulsified and then excreted trough a suction tube connected to a pressure reduced tank (Japanese Patent Application Laid-Open No. 6-197977). Another is that a synthetic resin transfusion tube is inserted from the anus, a solvent is injected to break and emulsify feces and then the feces are sucked and excreted (Japanese Utility Model Application Laid-Open No. 6-9554).

In the above means, however, since feces are forcibly moved inwardly by the pressure of the injected solution, they are made difficult to be broken.

An object of the present invention is to provide an excretion treatment apparatus solving the above problems.

### DISCLOSURE OF THE INVENTION

To achieve the above object, an excretion treatment apparatus of the present invention is provided with an insertion tool capable of injecting an irrigation solution and sucking feces at the same time.

### BRIEF DESCRIPTION OF THE DRAWINGS

Fig. 1 is a system diagram showing an excretion treatment apparatus based on an embodiment of the present invention;
Fig. 2 is a fragmentary sectional view showing a flared end of an insertion tool of Fig. 1;
Fig. 3 and Fig. 4 are sectional views showing modifications of the expanded end of the insertion tool, respectively;
Fig. 5 is a cross sectional view showing the relationship between the insertion tool and a nozzle;
Fig. 6 is a side elevational view showing the nozzle shown in Fig. 1; and
Fig. 7 and Fig. 8 are cross sectional views showing nozzles used to excretion treatment apparatuses of other embodiments, respectively.

### BEST MODE OF CARRYING OUT THE INVENTION

### [Embodiment 1]

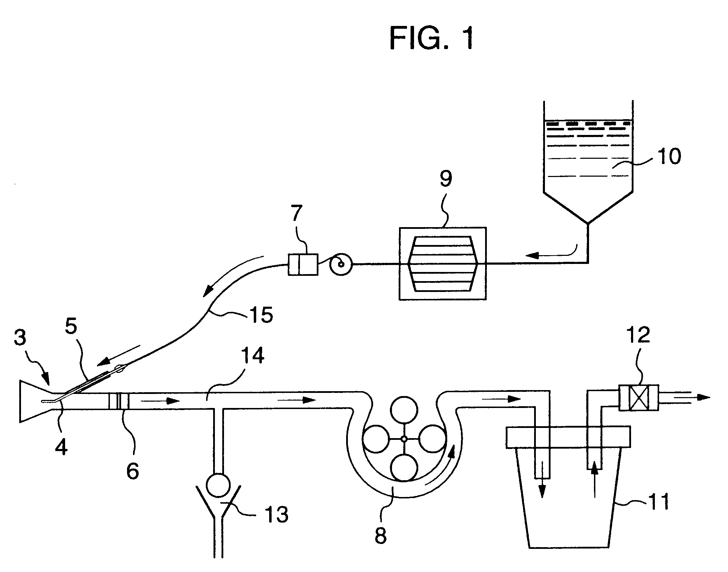

As shown in Fig. 1, an excretion treatment apparatus is composed of an insertion tool (3) to be inserted into the anus, an irrigation solution feed unit for feeding irrigation solution from an irrigation solution tank (10) to the insertion tool (3) through an irrigation solution pump (7), and a suction/discharge unit for sucking broken and emulsified feces to a storage vessel (11) by a suction pump (8) and discharging them.

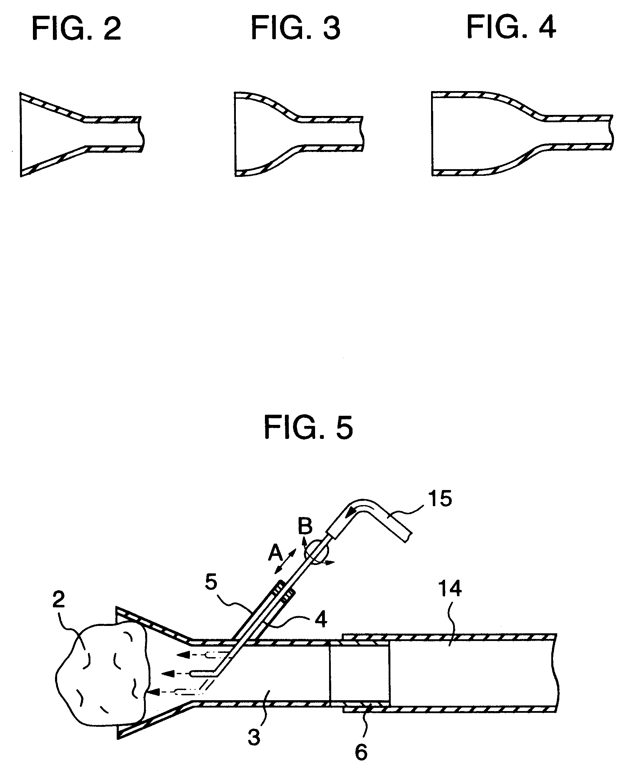

The cylindrical insertion tool (3) includes an irrigation nipple (5) made of an elastomer material and attached to the side thereof and a metal suction nipple (6) attached to an end thereof. The other end of the insertion tool (3) is flared as shown in Fig. 2.

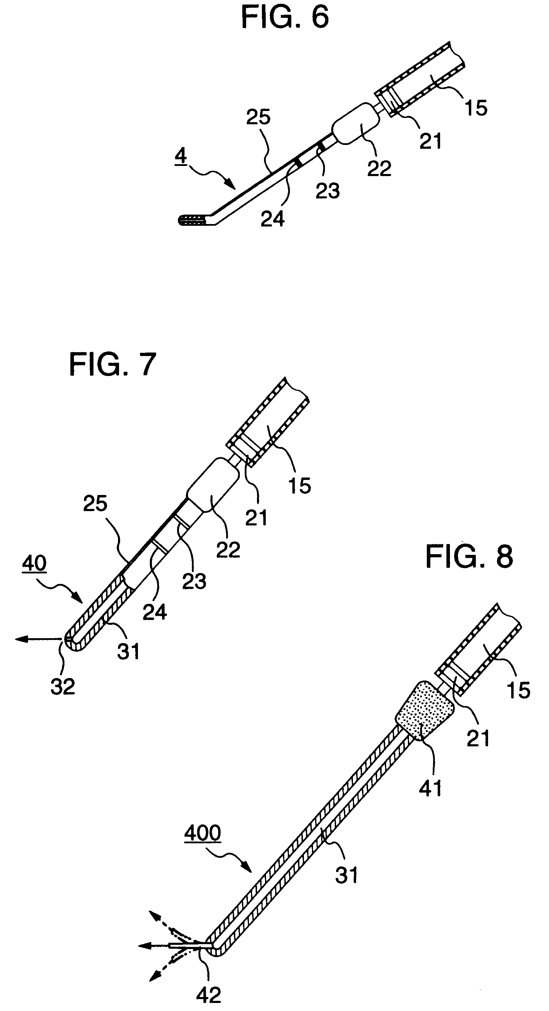

An irrigation nozzle (4) is inserted into the insertion tool (3) through an opening of the irrigation nipple (5). As shown in Fig. 6 in detail, the irrigation nozzle (4) is bent at a distal end thereof and an irrigation solution passage (31) is opened at a tip of the bent end. The irrigation nozzle (4) is provided at a proximal end thereof with a connector (21) and communicates with the irrigation solution tank (10) through an irrigation solution tube (15). The irrigation solution pump (7) is interposed between the irrigation nozzle (4) and the irrigation solution tank (10) to feed irrigation solution from the irrigation solution tank (10) to the insertion tool (3), that is, to the irrigation solution nozzle (4).

A suction nipple (6) of the insertion tool (3) communicates with the storage vessel (11) through a suction tube (14). A suction pump (8) is interposed between the suction nipple (6) and the storage vessel (11) for applying a negative pressure to an interior of the insertion tool (3).

The flared end of the insertion tool (3) evacuates the rectum and draws feces (2) into the insertion tool (3) by the negative pressure (refer to Fig. 5). For this purpose, at least the flared end of the insertion tool (3) is preferably made of a soft material, such as an elastomer material of silicon rubber or the like. Further, the shape of the flared end is not limited to the one shown in Fig. 2 and may be formed to other flared shapes as shown in Fig. 3 and Fig. 4.

A knob (22) is provided at the proximal end of the irrigation nozzle (4). The irrigation nozzle (4) can be moved along an axis thereof in the insertion tool (3) as shown by an arrow (A) of Fig. 5 or turned (swung) around the axis as shown by an arrow (B) of Fig. 5 through the knob (22). With this operation, the irrigation solution can be injected to omnidirection, or any arbitrary direction. Further, the knob (22) acts as a stopper for limiting an innermost position irrigation solution nozzle (4). As shown in Fig. 6, peripheral graduations (23), (24) and an axial graduation (25) are formed on the outer surface of the irrigation nozzle (4), the former graduations indicating where the irrigation nozzle (4) is located in the insertion tool (3) and the latter graduations indicating where the distal end opening of the irrigation nozzle (4) is directed. The alignment of the peripheral graduation (23) or (24) with the opening of the irrigation nipple (5) enables the distal end of the irrigation nozzle (4) to be positioned at a center in the radial direction of the insertion tool (3) or in the vicinity of the inner peripheral wall thereof, respectively. Further, the axial graduation (25) is aligned with the direction of the distal end opening of the irrigation nozzle (4), thereby confirming a direction to which the irrigation solution is injected.

Next, operation of the excretion treatment apparatus will be described.

An operator inserts the insertion tool (3) into the anus of a person being treated. Then, the operator inserts the irrigation nozzle (4) through the irrigation nipple (5) into the insertion tool (3). The irrigation tube (15) is connected to the connector (21) of the irrigation nozzle (4) and the suction tube (14) is connected to the suction nipple (6), respectively. Next, the irrigation solution pump (7) and the suction pump (8) are operated. The irrigation solution is injected from the irrigation nozzle (4) towards the feces (2) to break and emulsify the feces (2). The irrigation solution can be injected to any arbitrary direction in the insertion tool (3) by the operation of the knob (22) as shown in Fig. 5.

The irrigation solution is preferably injected at between 2 m/sec. and 10 m/sec. When the injection speed is low, feces cannot be sufficiently broken, whereas when the injection speed is too high, there is a possibility that the intestinal wall may be damaged. Further, a flow rate of the injected irrigation solution is preferably between 50 mℓ/min. and 1 ℓ/min. When the flow rate is small, there is a possibility that a flow passage is clogged because the fluidity of feces is made bad. When the flow rate is large on the contrary, the waste stored in the storage vessel 11 must be often disposed of.

To obtain a relatively small flow rate and high injection speed, it is preferable to feed the irrigation solution in the form of a pulsating flow or a pulse flow. Consequently, a piston type pump or a bellows type pump is preferable as the irrigation solution pump (7) of the present invention.

Further, it is preferable to preheat the irrigation solution to a temperature near to a body temperature by means of a preheater (9) or the like in order to prevent the bowels from being excessively excited and the body temperature from being lowered. The alternative thereto is that hot water is added to the irrigation solution tank (10). When the preheater is used, water pipes may be directly connected to the preheater in place of the irrigation solution tank 10. Water or a water solution mixed with an electrolyte and/or a surfactant material such as polyethylene glycol or the like may be used as the irrigation solution.

Feces broken and emulsified by the injection of the irrigation solution is sucked to the storage vessel (11) together with the irrigation solution by the suction pump (8) and stored therein. There is a possibility that the feces in the rectum are forcibly moved into the bowels far away from the irrigation nozzle (4) by the injection of the irrigation solution and cannot be sufficiently broken. To prevent this phenomenon, the suction pump (8) is operated simultaneously with the injection of the irrigation solution in the present invention, so that the feces are drawn near to the flared end of the insertion tool (3) as shown in FIG. 5. Accordingly the feces are effectively broken under the high pressure of the injected irrigation solution. Most suitable as the suction pump is a roller pump which can securely execute sucking operation without the need of priming water even at a low flow rate and has no mechanical parts in contact with the waste solution.

It is preferable that the suction tube (14) for guiding the broken feces to the storage vessel (11) is provided with a uniform inner diameter without steps and is made of soft polyvinyl chloride or silicon rubber so that the interior of the tube can be visually observed. However, the suction tube is not limited to the above materials.

Suction strength is adjusted by the number of rotation of the suction pump (8). When suction is excessively strong, since a strong negative pressure is created in the bowels, the intestinal wall is contracted and the feces cannot be sucked. Further, there is a possibility that the intestinal wall is sucked into the insertion tool (3) and damaged. To prevent the possibility, the embodiment is provided with a release valve (13) at the midway of the suction tube (14) which would be opened at predetermined negative pressure to prevent the pressure in the suction tube (14) from being lowered below it (to the greater negative pressure). The predetermined negative pressure is preferably between -10 cm H₂O and -50 cm H₂O.

The sucked feces and irrigation solution are stored in the storage vessel (11). Gases in the storage vessel (11) are deodorized by a deodorant column (12) filled with active carbon, silicate minerals or the like and then discharged to the outside air. The contents stored in the storage vessel (11) are disposed of to a toilet or the like and the storage vessel (11) is cleaned and reused. A bag may be placed in the storage vessel 11, into which the faces are stored, and the feces may be disposed of together with the bag.

### [Embodiment 2]

In this embodiment, an irrigation nozzle (40) as shown in Fig. 7 is employed. The irrigation nozzle (40) has a distal which is straight without being bent. An irrigation solution passage (31) is opened to an injection port (32) obliquely formed at the distal end portion of the nozzle. An axial graduation (25) is formed to be in alignment with the injection port (32). According to this embodiment, the irrigation nozzle (40) can be easily inserted into the irrigation nipple (5) and a sealing effect can be improved.

### [Embodiment 3]

In this embodiment, an irrigation nozzle (400) as shown in Fig. 8 is employed. The irrigation nozzle (400) is straight and has a soft nozzle (42) obliquely extending from a distal end of the nozzle. The soft nozzle (42) is a tube made of a soft and elastic elastomer material such as silicon rubber or the like. The soft nozzle (42) is vibrated at an end thereof by the pulsating flow of the irrigation solution from the irrigation solution pump (7), thereby naturally injecting the irrigation solution to various directions, and thus the irrigation nozzle (400) need not to be moved. A seal plug (41) is provided at a portion of the irrigation nozzle (400) so that the irrigation nozzle would be positioned at a predetermined position within the insertion tool (3) and is fixed to an opening of the irrigation nipple (5) so that the soft nozzle (42) is directed to the feces. According this, the irrigation solution can be injected to any arbitrary direction and a sealing effect can be secured between the irrigation nozzle (400) and the irrigation nipple (5).

The excretion treatment apparatus of the present invention draws the feces to the distal end portion of the insertion tool by means of the suction pump, injects irrigation solution to the feces by means of the irrigation solution pump to break and emulsify them, and sucks the feces by means of the suction pump. Different from conventional enema and bowel washing, the irrigation solution is injected only to the lower digestive tract and after the feces are broken, the irrigation solution is sucked by the suction pump together with the broken feces. As a result, the irrigation solution remaining in the bowels is reduced, thus there is almost no possibility that the irrigation solution remaining in the bowels leaks.

When feces in the near part is sucked, since feces in the inner part are sequentially pushed out by the negative pressure generated by the suction pump and the meandering motion of the intestine, it suffices only to insert the insertion tool to a portion about 3 cm to about 8 cm from the anus.

### Industrial Applicability

Feces can be very conveniently and safely excreted by the use of the excretion treatment apparatus of the present invention at a low cost through a closed system, thus the apparatus is very useful to medical care industries.

## Claims

1. An excretion treatment apparatus for breaking, emulsifying, sucking and discharging feces, said apparatus comprising:
a tubular insertion tool having an irrigation nipple provided at a side thereof and a suction nipple provided at a one end thereof;
an irrigation nozzle inserted into said insertion tool through said irrigation nipple;
an irrigation solution feed unit including an irrigation solution pump and feeding an irrigation solution to said irrigation nozzle; and
a suction/discharge unit for communicating said suction nipple to a storage vessel, said unit having a suction pump disposed at a midway thereof, wherein both said pumps can be operated at the same time.

2. An excretion treatment apparatus according to Claim 1, wherein at least the other end of said insertion tool is made of elastomer and said the other end of said insertion tool is flared.

3. An excretion treatment apparatus according to Claim 1 or claim 2, wherein said irrigation nozzle is tubular and, bent at a distal end portion thereof, and can move in the direction of an axis of said irrigation nozzle as well as turn around said axis.

4. An excretion treatment apparatus according to Claim 1 or claim 2, wherein said irrigation nozzle is straight and tubular with a closed distal end thereof, and has an opening directed to cross an axis of said irrigation nozzle, and can move in the direction of said axis of said irrigation nozzle as well as turn around said axis.

5. An excretion treatment apparatus according to Claim 1 or claim 2, wherein said irrigation nozzle is tubular with a closed distal end thereof, and has a tube having elasticity in a direction crossing to the axis of said irrigation nozzle and projecting from said irrigation nozzle.
